# FASCICULE DE BREVET EUROPEEN

(11) **EP 1 760 154 B1**
(45) Date de publication et mention de la délivrance du brevet: **11.08.2010**
(21) Numéro de dépôt: 06018991.7
(22) Date de dépôt: 25.07.1996
(51) Int. Cl.: C12N 15/86, C12N 5/10, A61K 48/00

(54) **Séquence de rétroéléments naturels ou synthétiques ayant pour fonction de permettre l'insertion de séquences nucléotidiques dans une cellule eucaryote**
Sequenzen von natürlischen oder synthetischen Retroelementen, die die Insertion von nukleotiden Sequenzen in eukaryotische Zellen erlauben
sequences from natural or synthetic retroelements having the function of allowing the insertion of nucleotide sequences in a eukaryotic cell

(30) Priorité: 07.08.1995 FR 9509587
(43) Date de publication de la demande: 07.03.2007
(62) Demande divisionnaire de: 96927085.9
(73) Titulaire: INSTITUT PASTEUR, 75724 Paris Cedex 15 (FR)
(72) Inventeur: Choulika, André, 75015 Paris (FR); Nicolas, Jean-François, 78590 Noisy le Roi (FR)
(74) Mandataire: Desaix, Anne

(56) Documents cités:
- EP-A- 0 300 422
- EP-A- 0 336 822
- WO-A-92/07943

## Description

La présente invention concerne un vecteur rétroviral caractérisé en ce qu'il comprend un polynucléotide comprenant (a) la séquence LTR3' et LTR5' d'un retroélément naturel ou synthétique ; et (b) une séquence d'insertion comprenant une séquence de nucléotides d'intérêt ainsi que, pour un système de recombinaison donné, une unique séquence de reconnaissance d'une recombinase, ladite séquence d'insertion étant insérée dans ladite LTR3' ou ladite LTR5', de façon à permettre l'insertion de ladite séquence d'intérêt dans le génome d'une cellule hôte, sans que soient insérées des séquences provirales qui ne sont plus nécessaires après l'intégration de la séquence d'intérêt dans le génome de la cellule hôte, l'expression de la recombinase reconnaissant ladite séquence de reconnaissance n'étant pas associée directement audit vecteur.

Les récents progrès dans l'utilisation des rétrovirus en tant que vecteurs de gènes entrent dans trois catégories : (i) amélioration des lignées cellulaires d'encapsidation, (ii) manipulation du tropisme des protéines de l'enveloppe, (iii) expression de gènes multiples. La modification de la structure du provirus intégré n'a pas encore été très exploitée parce que cette dernière contient certains éléments essentiels agissant en *cis*. Ces éléments sont la source de multiples problèmes.

La demande WO92/07943 décrit des vecteurs rétroviraux, comprenant un site d'insertion pour y inclure des gènes d'intérêt, qui permettent une expression à un niveau élevé du gène d'intérêt qui y est inséré, par exemple en tirant parti de séquences régulatrices rétrovirales. En revanche, la demande ne vise pas de vecteurs permettant "intégration des gènes d'intérêt tout en évitant celle de séquences provirales qui ne sont plus nécessaires après le mécanisme d'intégration, et à cet effet les vecteurs décrits ne comprennent pas de séquence de reconnaissance d'une recombinase.

La demande EP0300422 décrit une méthode pour préparer des vecteurs rétroviraux recombinants en utilisant le système CRE/loxP, reposant sur un premier vecteur « générique », comportant un site IoxP inséré dans son génome mais ne comportant pas de séquence nucléotidique d'intérêt, et un second vecteur comprenant une séquence d'intérêt et un site IoxP mais ne comprenant pas de séquence de rétroéléments (tels que des LTR). De plus, ces deux types de vecteurs ne comprennent pas une séquence d'intérêt et un site de reconnaissance d'une recombinase, insérées dans un LTR.

Du point de vue de la structure, le système de vecteur rétroviral est construit sur deux éléments : les gènes transcomplémentants (*gag, pol* et *env*), et les séquences agissant en cis (U3, R, U5, la piste polypurine (PPT), le site de liaison de l'amorce (PBS) et le signal d'encapsidation et de dimérisation).

Les gènes transcomplémentants sont incorporés dans des cellules transcomplémentantes. Ils ne sont pas transférés vers les cellules cibles.

Les séquences agissant en *cis* sont incorporées dans des vecteurs rétroviraux. La plupart d'entre elles sont transférées vers les cellules cibles infectées et sont intégrées dans le provirus recombinant L'élimination de n'importe laquelle de ces séquences agissant en *cis* aboutit à un système rétroviral non-fonctionnel. Le signal d'encapsidation est nécessaire pour l'encapsidation du génome rétroviral dans des particules virales. PBS, PPT et R sont requis pour la transcription inverse. Les séquences U3 et U5 sont indispensables pour la transcription inverse et pour l'intégration du produit rétroviral introduit dans la cellule.

Une fois le provirus intégré, ces séquences ne sont plus nécessaires pour l'expression du gène. Au contraire, ces séquences peuvent même être la cause de nombreux problèmes. (i) Une interférence transcriptionnelle peut résulter de la présence du promoteur fort dans les séquences U3. (ii) Le PBS peut agir comme un élément inhibiteur en *cis* pour les promoteurs internes. Par exemple, dans les cellules pluripotentes, le PBS est une séquence de reconnaissance pour un répresseur fort et en plus il agit comme un élément inhibiteur. (iii) La séquence U3 contient au moins un des éléments de régulation négative situés dans les activateurs à distance répétés directs entre -345 et -306. (iv) La LTR3' (long terminal repeat) peut activer des gènes flanquant des cellules avec parfois des conséquences délétères pour la cellule. (v) L'ARN exprimé à partir du promoteur de la LTR5' contient le signal d'encapsidation, et peut, par conséquent, être récupéré dans une particule rétrovirale. Cet ARN peut se recombiner avec un ARN cellulaire ou l'ARN rétroviral d'un phénotype, aboutissant à la récupération d'un rétrovirus doté de nouvelles propriétés (créant un danger biologique potentiel). Ces problèmes n'ont pas encore été entièrement surmontés puisque tous ces éléments sont nécessaires pour la réplication et l'insertion du virus dans le génome.

Dans le contexte de la présente invention, les inventeurs ont développé un vecteur rétroviral conçu de façon à permettre l'élimination d'une grande partie des séquences provirales qui ne sont plus nécessaires après l'intégration d'un provirus dans une cellule hôte. Plus particulièrement, ce vecteur rétroviral permet l'intégration d'une seule séquence LTR, d'un rétrotransposon, ou une séquence comprenant une région U3, R ou U5 dans le génome d'une cellule hôte.

L'invention concerne également les cellules hôtes, de préférence des cellules eucaryotes, obtenues après transfection par le vecteur rétroviral de l'invention.

La présente demande rapporte doncun vecteur retoviral caractérisé en ce qu'il comprend une séquence d'insertion incorporée dans une région pouvant être transférée dans une cellule cible et intégrée dans un provirus recombinant lors de l'infection de cette cellule cible par un rétrovirus comprenant cette séquence. Particulièrement, cette séquence d'insertion est incorporée dans une région agissant en cis, plus particulièrement, dans la région LTR3' ou LTR5' et de façon préférée dans la région U3 du LTR3' ou U5 du LTR5' de cette séquence rétrovirale. Cette séquence d'insertion comprend une séquence de nucléotides d'intérêt pouvant être intégrée dans le génome d'une cellule hôte ainsi qu'une séquence de reconnaissance pour une recombinase. De façon préférée, la séquence d'insertion ne contient qu'une seule séquence de reconnaissance située avec la séquence d'intérêt, en amont ou en aval par exemple, bien que ce dernier paramètre ne soit pas absolument nécessaire. La séquence de nucléotides d'intérêt peut être située en aval de la séquence de reconnaissance dans la mesure où cette dernière est comprise dans la région transférée dans une cellule cible lors de l'infection de cette cellule cible par un rétrovirus contenant la séquence rétrovirale. La longueur maximale de la séquence d'insertion que le vecteur rétroviral de l'invention contient se situe normalement entre 0,5 et 10 kb. Le vecteur rétroviral, qui peut se présenter sous forme de rétrovirus recombinant, ne contient qu'une seule séquence de reconnaissance incorporée dans la région pouvant être transférée à une celule cible.

L'ADN de l'invention permet donc l'insertion, par l'entremise d'un vecteur rétroviral, de séquences de nucléotides d'intérêt dans des cellules hôtes, par exemple de type eucaryote, sans que soient insérées des séquences provirales qui ne sont plus nécessaires après l'intégration des séquences d'intérêt dans le provirus.

Le terme « séquence de nucléotides d'intérêt » utilisé ci-dessus fait référence à des séquences à insérer dans le génome de cellules hôtes pour ainsi permettre à celles-ci de produire des molécules d'intérêt, plus particulièrement en thérapie ou pour la vaccination. Ces séquences d'intérêt incluent entre autres des gènes, des séquences d'ADN ou d'ARN codant soit pour des protéines (hormones, immunoglobulines enzymes ou autres) lorsque les vecteurs rétroviraux de l'invention sont utilisés en thérapie génique, soit des protéines humaines ou non humaines (telles des protéines virales) lorsqu'il s'agit d'utiliser les vecteurs rétroviraux de invention dans le cadre de protocoles de vaccination. Les séquences nucléotidiques d'intérêt peuvent aussi être constituées en partie d'éléments de régulation (i.e. promoteur, activateur) homologues ou hétérologues à la cellule hôte d'une part et d'autre part de séquences codant pour tout ou partie d'un ou plusieurs gènes ou ADN complémentaire. De plus, les séquences nucléotides d'intérêt peuvent aussi coder un ARN antisens ou une séquence ribozyme.

Les applications possible du vecteur rétroviral de la présente invention sont multiples. Le vecteur rétroviral de l'invention est utilisée soit simplement pour l'insertion de séquences de nucléotides dans des cellules hôtes telles des cellules eucaryotes dans un environnement qui favorise une meilleure expression de ce gène, soit en thérapie génique soit en vaccination. A titre d'exemple, la séquence d'intérêt décrite dans Nature Medicine, Volume No. 7, Juillet 1995 correspondant à l'insérat des plasmides pMEPV_{H}/pMEPV_{L} ou pMEPV_{H}/pREPV_{K} peut être utilisée et le produit d'expression in vivo peut être un élément d'une composition thérapeutique.

Le vecteur rétroviral de l'invention est obtenu par la transfection d'une lignée cellulaire virale transcomplémentante avec une séquence de rétroéléments de l'invention se présentant de façon préférée sous forme de plasmide et comprenant de façon préférée dans une de ses LTR et plus particulièrement dans sa LTR de droite une séquence de nucléotides d'intérêt ainsi qu'une séquence de reconnaissance. Une fois la transfection de la lignée cellulaire virale effectuée, on obtient un vecteur rétroviral qui peut être utilisé pour l'infection d'une cellule hôte dans laquelle on souhaite intégrer la séquence de nucléotides d'intérêt.

Le plasmide défini ci-dessus, ainsi que la méthode de transfection de la lignée cellulaire par ledit plasmide, entrent également dans le cadre de la présente invention.

L'invention concerne également une méthode ou procédé d'introduction de séquences de nucléotides d'intérêt dans une cellule hôte en culture, telle une cellule eucaryote. La méthode est caractérisée en ce qu'elle comprend l'introduction d'une séquence correspondant à des rétroéléments tels que les régions U3, U5 et/ou R dans une cellule eucaryote en culture , ladite séquence étant liée à une séquence nucléotidique d'intérêt. Cette méthode est caractérisée par une étape d'infection d'une cellule eucaryote par un vecteur rétroviral contenant la séquence rétrovirale de l'invention qui comprend la séquence de nucléotides à introduire dans la cellule eucaryote, dans des conditions permettant l'intégration dans le génome cellulaire de la cellule d'une seule LTR du vecteur rétroviral contenant la séquence à introduire dans le génome de la cellule hôte et une seconde étape d'expression de la séquence d'intérêt puis les éléments du produit de celle-ci à partir de la cellule recombinée.

La demande rapporte également une méthode de traitement médical comprenant l'administration d'une quantité efficace d'un vecteur rétroviral de l'invention comprenant une séquence de nucléotides comportant un gène susceptible d'être intégré dans le génome de certaines cellules cibles du malade. Les vecteurs rétroviraux de la présente invention peuvent donc être utilisés en thérapie génique.

La demande rapporte également un hôte cellulaire ayant intégré dans son génome une structure provirale comprenant une seule région LTR d'un rétrovirus. Cette séquence LTR comporte une copie unique d'une séquence de nucléotides d'intérêt. Plus particulièrement, l'hôte cellulaire de est une cellule eucaryote et la structure provirale est caractérisée en ce qu'elle est essentiellement libre de séquence PBS, de signal d'encapsidation et de dimérisation et/ou de PPT. La demande rapporte aussi les hôtes cellulaires recombinants contenant la séquence unique LTR ou une partie de celle-ci et la séquence d'intérêt.

La demande rapporte également l'utilisation du vecteur rétroviral de l'invention dans la production dans des cellules hôtes de protéines codées par une séquence de nucléotides d'intérêt comprise dans cet ADN rétroviral ou dans le traitement médical d'un patient visant à intégrer une séquence de nucléotides d'intérêt dans le génome de cellules visées.

Plus particulièrement, le vecteur rétroviral de l'invention permet également le transfert de séquences de nucléotides codant pour des anticorps dans des cellules hôtes. On parle alors d'immunisation extracellulaire puisque les anticorps générés à partir de ces séquences produisent leur effet à l'intérieur des cellules les ayant intégrées. Parmi les séquences de nucléotides codant pour des anticorps pouvant être intégrées dans des cellules hôtes en utilisant la séquence rétrovirale et le vecteur rétroviral de l'invention, on peut mentionner, à titre d'exemple non limitatif, les anticorps reconnaissant des protéines d'enveloppe de virus HIV, particulièrement les protéines gp.120 du virus HIV-1 et du virus HIV-2 ainsi que des anticorps permettant de bloquer la transcriptase inverse. De tels anticorps sont décrits entre autres dans la publication de Maciejewski et al (Nature Medicine, Volume No 7, juillet 1995). Le vecteur rétroviral de l'invention permettant une intégration plus efficace de ces séquences de nucléotides d'intérêt, l'efficacité thérapeutique de cette approche en est d'autant améliorée.

La demande rapporte donc l'utilisation de vecteurs rétroviraux comprenant une séquence de nucléotides d'intérêt codant pour un anticorps reconnaissant une protéine ou un fragment d'une protéine de virus HIV, ainsi qu'une séquence de nucléotides de reconnaissance, ces deux séquences de nucléotides étant situées dans une région pouvant être transférée dans des lymphocytes humains à la suite d'une infection. De façon préférée, ces deux séquences sont situées dans la région LTR3' du vecteur rétroviral.

L'invention concerne également une méthode ou procédé d'expression d'une molécule codée par une séquence de nucléotides d'intérêt dans une cellule hôte en culture. La méthode comprend :
- l'infection de la cellule hôte par un vecteur rétroviral de l'invention comprenant la séquence de nucléotides d'intérêt ;
- la croissance de la cellule hôte dans des conditions permettant l'expression de la séquence de nucléotides d'intérêt ; et
- l'obtention de la molécule désirée.

Les vecteurs rétroviraux de l'invention peuvent être utilisés de plusieurs façons pour permettre l'intégration de la séquence de nucléotides d'intérêt désirée. En fait, les inventeurs ont démontré qu'il n'était pas nécessaire d'associer l'expression de la recombinase directement au vecteur rétroviral, bien que les rendements d'intégration soient supérieurs lorsqu'une séquence nucléotidique codant pour la recombinase est partie intégrante du vecteur.

Un vecteur rétroviral comprenant dans la région LTR3' ou LTR5' du vecteur rétroviral une première séquence de nucléotides comprenant une séquence de nucléotides d'intérêt ainsi qu'une séquence de reconnaissance située en aval ou en amont de la séquence d'intérêt, ainsi qu'une deuxième séquence de nucléotides identique à la première séquence de nucléotides insérée dans la région LTR5' du vecteur, est introduit dans le génome d'une cellule hôte par infection. Un plasmide comprenant une séquence de nucléotides codant pour une recombinase susceptible de reconnaître la séquence de reconnaissance est également introduite dans la cellule par transfection. L'expression de la recombinase par transfection transforme la structure d'un provirus à deux LTR à un provirus à une seule LTR. Dans ce type de réaction, environ 50% des transformants stables contiennent le provirus modifié. Ce résultat partiel peut s'expliquer par l'expression éphémère du plasmide comprenant la séquence de nucléotides codant pour la recombinase après la transfection, aboutissant à une recombinaison sans intégration.

On utilise une séquence rétrovirale comprenant une séquence nucléotidique comprenant une séquence d'intérêt ainsi qu'une séquence de reconnaissance située en aval ou en amont de cette séquence d'intérêt. La séquence complète est insérée dans la région LTR3' d'un rétrovirus. Le vecteur rétroviral ainsi obtenu est ensuite utilisé pour infecter une lignée cellulaire qui exprime constitutivement ou par induction une recombinase susceptible de reconnaître la séquence de reconnaissance. L'efficacité d'un tel système est élevée surtout lorsque le gène codant pour la recombinase est exprimé dès le début de l'infection rétrovirale.

Dans une des réaiisations divulguées dans la demande les inventeurs ont utilisé le système de recombinase *Crelox* spécifique du site du bactériophage P1. Le site de reconnaissance *LoxP* a été inséré dans la LTR3' en amont de la séquence de nucléotides d'intérêt dans un vecteur de type ΔEnh, et le gène codant pour la recombinase *cre* entre les deux LTR. Dans la lignée cellulaire productrice, la protéine Cre est exprimée à partir de la construction plasmidique provirale. Toutefois, puisque le plasmide ne contient qu'une seule cible *IoxP,* il n'est pas recombiné par la protéine Cre. Après l'infection, le site *IoxP* est dupliqué de la LTR3' dans la LTR5'. Ainsi, Cre recombine les deux sites *IoxP* répétés directs, ce qui se traduit par la délétion de toutes les séquences comprises entre les deux *IoxP,* y compris le PBS, le signal d'encapsidation et de dimérisation et la PPT. Ne reste alors intégré dans le génome cellulaire qu'une seule LTR contenant le gène rapporteur. Le gène *cre* est également perdu lors de la recombinaison.

Dans tous les modes de réalisation préférentiels décrits ci-dessus, la séquence de nucléotides d'intérêt, qui est généralement une séquence étrangère au rétrovirus, est introduite de façon avantageuse dans la région U3 de LTR3' de la séquence rétrovirale. En effet, la possibilité d'introduire des séquences, voire des unités transcriptionnelles complètes, dans la région U3 de LTR3' est très bien documentée. On peut cependant envisager l'introduction de séquences d'intérêt ailleurs que dans la région U3 de LTR3' du vecteur rétroviral. Des exemples incluent la région U5 du LTR5'. De plus, bien qu'il soit préférable que le site de reconnaissance soit situé immédiatement en aval ou en amont de la séquence de nucléotides d'intérêt, on peut également positionner cette séquence à une distance appréciable qui peut varier entre 32nt et 4.5 Kb sans affecter l'intégration finale de la séquence d'intérêt à la cellule visée.

La quantité de produits génétiques étrangers introduite dans la région U3 de l'ADN viral peut être élevée et dans certains cas supérieure à 4 kpb. Ces séquences peuvent même correspondre à une unité transcriptionnelle complète avec un promoteur indépendant, ce qui démontre la polyvalence de ces vecteurs.

La possibilité d'introduire des quantités considérables de produits génétiques étrangers dans la 3' LTR du vecteur rétroviral de l'invention fait de ce vecteur rétroviral un système de choix pour l'insertion de séquences de gènes dans des cellules, plus particulièrement dans des cellules eucaryotes. Plusieurs types de cellules eucaryotes peuvent ainsi être transformées. Parmi les cellules eucaryotes pouvant être utilisées dans le contexte de la présente invention, notons particulièrement les types de cellules suivants : NIH 3T3, BRL, HM1, D3, PLL4, LT.

Dans une des réalisations particulières de la présente invention, l'insertion d'une séquence de nucléotides codant pour un marqueur est prévue dans la région LTR3' du vecteur rétroviral. La présence de ce marqueur permet de prouver l'insertion du produit nouveau.

A titre d'exemples non limitatifs, les marqueurs suivants peuvent être utilisés : LacZ, GFP (green fluorescent protein), CD9, PAL (alkaline phosphatase) et HRP (Horse Radish Peroxydase).

Bien que le système de recombinase Crelox constitue un système de choix pour le vecteur rétroviral de l'invention, d'autres systèmes de recombinase peuvent également être utilisés. A titre d'exemples non limitatifs, les systèmes de recombinaison suivants sont utilisables : le système de levure FLP (dit « Flip ») et le système bactérien R.

Les vecteurs rétroviraux de l'invention fournissent donc un moyen de transfert efficace d'ADN dans des cellules hôtes. L'intégration provirale est précise et ne provoque pas de réarrangement chromosomique. La conception des vecteurs duplicateurs partait du principe que la transposition du gène dans la LTR5', à l'extérieur de l'unité transcriptionelle rétrovirale, améliore son expression.

Bien que la présence du signal d'encapsidation rende possible la génération d'un virus compétent pour la réplication par recombinaison entre les séquences du rétrovirus et du provirus endogènes, que la présence de PPT et d'autres éléments en cis rendent la transposition possible et que la présence des séquences PBS puisse avoir des effets négatifs sur l'expression du transgène, tous ces évènements sont très improbables lorsqu'on utilise un vecteur rétroviral selon l'invention puisque toutes ces séquences virales sont délétées.

Le système des sLTR est un concept nouveau dans la construction des rétroéléments en général. Il existe de nombreux rétrovirus dont le spectre d'hôte de la particule présente un intérêt mais pour lesquels la conception de vecteurs nécessite la présence de certaines séquences *cis* ou *trans* actives différentes de celles qui ont été citées précédemment qui permettent un assemblage correct du virion et lui permettent d'être infectieux, comme par exemple les virus humains VIH, HTLV ou le virus de chèvre CAEV. Une fois intégrés, les éléments *cis* ou *trans* régulateurs deviennent inutiles pour le gène transduit mais ont des effets pathogènes pour la cellule et augmentent significativement les probabilités de la résurgence d'un virus sauvage. La nécessité de ce type de séquence pour ce type de rétrovirus les rend pratiquement inutilisables pour un usage en tant que vecteurs. L'application du système sLTR de la présente invention pour ces rétrovirus permet d'envisager avec plus de sécurité et plus de fiabilité transcriptionnelles un usage comme vecteurs pour la transduction de gènes.

La présente invention sera nettement décrite en référence aux exemples suivants non limitatifs et qui font référence aux figures suivantes :
- la figure 1A représente la structure du plasmide pMloxPL ;
- la figure 1B représente la structure du provirus MloxPL résultant de l'infection du rétrovirus MloxPL ;
- la figure 1C représente un provirus après la recombinaison induite par cre utilisant les deux sites IoxP à l'intérieur des LTR ;
- la figure 1D représente une analyse par « Southern blot » de l'ADN cellulaire provenant de fibroblastes NIH3T3 infectées par MloxPL;
- la figure 1E présente une analyse par « Southern Blot » de l'ADN cellulaire provenant du clone NIH3T3 MloxPL.1 transfecté par le plasmide pMC1-Cre;
- la figure 2A représente la structure et l'analyse moléculaire du provirus MloxPL résultant de l'infection du rétrovirus MloxPL et de la recombinaison induite par Cre utilisant les deux sites IoxP à l'intérieur des LTR;
- la figure 2B représente les résultats d'une analyse par « Southern btot » de l'ADN cellulaire provenant de fibroblastes NIH 3T3 Cre.1 infectés par MloxPL avec digestion par l'endonuclease de restriction Bcll ;
- la figure 2C représente les résultats d'une analyse par « Southern blot » de l'ADN cellulaire provenant de fibroblastes NIH 3T3 Cre.1 infectés par MloxPL avec digestion par les endonucleases ECORV ;
- la figure 3A représente la structure du plasmide pMCreloxPL ;
- la figure 3B représente un diagramme du 3' sLTR de quelques réalisations préférentielles du vecteur rétroviral de l'invention :
- la figure 3C représente les résultats d'une analyse par « Southern blot » de l'ADN cellulaire provenant de fibroblastes NIH 3T3 infectées par MCreloxPL par digestion par l'endonuclease de restriction Bcll ;
- la figure 3D représente les résultats d'une analyse par « Southern blot » de l'ADN cellulaire provenant de fibroblastes NIH 3T3 infectées par MCreloxPL par digestion par l'endonuclease de restriction EcoRV ;
- la figure 3E représente les résultats d'une analyse par « Southern blot » de l'ADN cellulaire provenant de fibroblastes NIH 3T3 infectées par MCreloxPL par digestion par l'endonuclease de restriction Kpnl ;

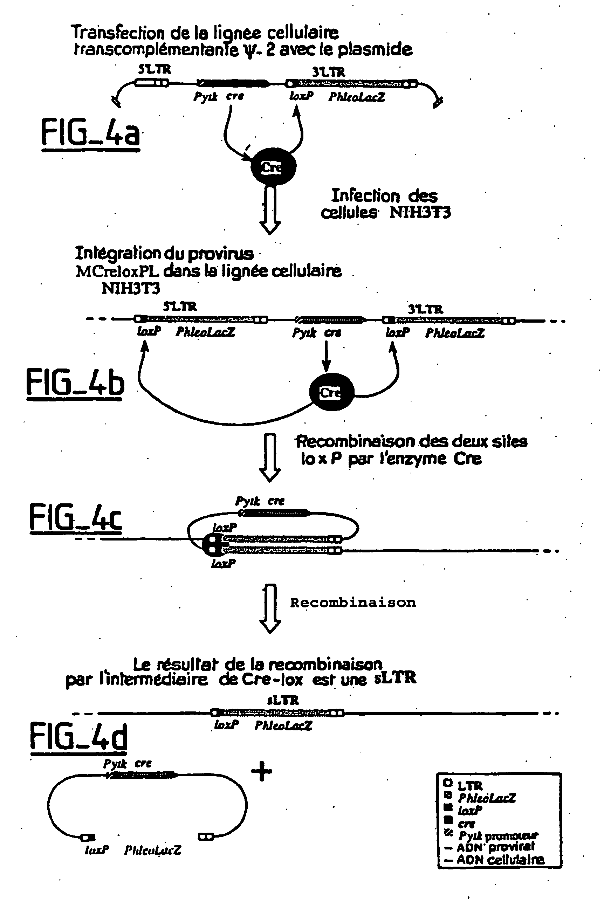

- la figure 4 représente un schéma du mécanisme de la génération de sTLR avec le vecteur rétroviral McreloxPL

### EXEMPLES

### Nomenclature

La nomenclature des différents vecteurs et des différentes cellules a été établie comme suit : le p désigne le vecteur plasmidique (par exemple, pMloxPL) ; les noms avec un Ψ2 (déposée le 10/3/1993 à l'ECAC sous le numéro 93031002) désignent la lignée cellulaire du virus sauvage auxiliaire Ψ2 transfectée avec ce vecteur (par exemple, Ψ2-MloxPL) ; les noms sans p désignent les virus produits par les cellules du virus sauvage auxiliaire (par exemple, MloxPL) ; le nom d'une lignée cellulaire suivi du nom du virus indique que la lignée cellulaire contient un provirus résultant d'une infection (par exemple, NIH3T3MloxPL).

### Culture et sélection des cellules

Les lignées cellulaires établies NIH 3T3 (fibroblastes de souris) et Ψ 2 (lignée cellulaire d'encapsidation du rétrovirus écotrope de la souris) sont référencées dans (7) et (13). Elles sont cultivées dans un DMEM (milieu de Eagle modifié par Dulbecco) à teneur élevée en glucose (4,5 g/l) complété avec du sérum de veau foetal à 5 %. Les cellules sont incubées à 37°C dans une atmosphère humidifiée, contenant 12% de CO₂. Du G418 est ajouté dans le milieu approprié à une concentration de 600 mg/ml. Le clonage de colonies résistant au G418 a été réalisé par pipettage de colonies individuelles et isolement dans une boîte de culture distincte. Le clonage des cellules infectées est réalisé par dilution limitante 48 heures après l'infection.

### Transfection, infection, coloration des cellules et analyse des acides nucléiques par "Southern blot"

Des précipitations par phosphate de calcium ont été effectuées comme décrit dans (12). Le surnageant contenant le virus a été utilisé pour infecter des cellules NIH 3T3 en présence de 5 µg/ml de polybrène, comme décrit précédemment dans (37). Des hybridations par "Southern blot" ont été préparées par le procédé en référence (6). L'activité β-galactosidase a été mise en évidence par coloration au X-gal, comme décrit précédemment dans (38).

### Détection de LacZ par PCR

La PCR est effectuée sur 1 µg d'ADN cellulaire génomique dans une réaction de 40 µl (Tris-HCl 50 mM (pH9), 150 µg/ml d'albumine de sérum bovin, (NH₄)₂ OS₄ 16 mM, Mgcl₂ 7 mM, 250 µM pour chaque dNTP, 1,25 U Taq DNApol (USB), 0,078 U Vent(exo+) DNApol (N.E. Biolabs)). Des amorces ont été ajoutées à une concentration finale de 0,25 µM (pour 20 mères). La réaction de la PCR a été chauffée à 80 °C pendant 5 minutes avant le début. 35 cycles (94°C, 55 min.; 59°C, 30 s; 70°C, 3 min. 30 s) avec les amorces suivantes : 5'-GCATCGAGCTGGGTAATAAGCGTTGGCAAT-3' et 5'-GACACCAGACCAATGGTAATGGTAGCGAC-3' pour la détection de *LacZ* et 5'-GGACTGGGTGGCTTCCAACTCCCAGACAC-3' et 5'-AGCTTCTCATTGCTGCGCGCCAGGTTCAGG-3' pour la détection du RAP-SYN endogène de souris, comme témoin interne. Les produits de réaction de la PCR ont été analysés par électrophorèse sur gel.

### Exemple 1 - Construction du vecteur rétroviral pMloxPL et transfection de lignées cellulaires

### a) Construction du vecteur pMloxPL

pMloxPL résulte du plasmide pG-MPL (décrit dans Choulika *et al,* 1995), dans lequel le site de reconnaissance *loxP* est inséré à l'intérieur du site *Nco* I dans un adaptateur de liaison (5'-CATGCATATAACTTCGTATAGCATACATTATACGAAGTTATC-3' et 5'-CATGGATAACTTCGTATAATGTATGCTTATCGAAGTTATATG-3'), au lieu du site I-*Sce* I. La séquence du site *loxP* a été vérifiée par séquençage de l'ADN.

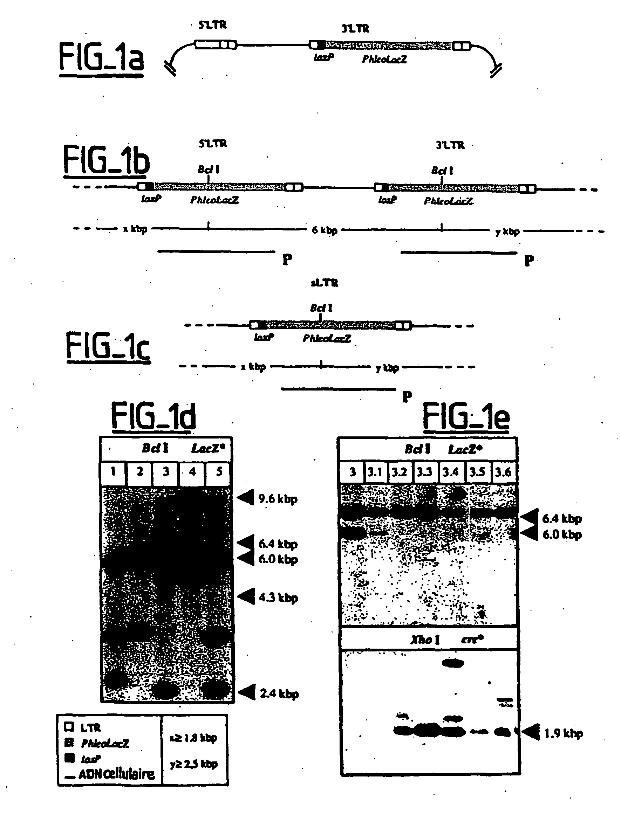

Le rétrovirus utilisé est illustré dans la Fig. 1 a. pMloxPL est construit à partir d'un provirus de leucémie murine de Moloney défectif (dépourvu du gène *gag*, *pol* et *env*) en insérant la séquence codant pour *PhleoLacZ* dans la région U3 de la LTR de droite à la place des séquences d'activation à distance. 5' issu du gène *PhleoLacZ* a été positionné entre le site accepteur d'épissage du gène *env* de MoMuLV et un site *loxP* long de 32 pb issu du bactériophage P1. Dans les cellules infectées, ce type de virus a la LTR qui contient le gène activée par un promoteur cellulaire flanquant par piégeage du promoteur.

### b) Transfection de lignées cellulaires avec le plasmide pMloxPL

Les lignées cellulaires produisant le virus ont été générées en transfectant le plasmide pMloxPL avec le plasmide de sélection pUSVneo dans la lignée cellulaire du virus sauvage auxiliaire Ψ2 qui exprime le virus sauvage auxiliaire écotrope défectif en encapsidation. Après sélection pour G418 (néomycine), des clones individuels sélectionnés sur leur activité β-galactosidase ont été testés pour titrer le virus. La titration a été effectuée en clonant des cellules NIH 3T3 immédiatement après infection par dilution limitante, et en détectant la présence du provirus contenant *LacZ* par PCR. Les lignées cellulaires productrices Ψ2-MloxPL.1 et Ψ2-MloxPL.2 ont montré un titre sur la lignée cellulaire NIH3T3 de respectivement 2,5 x 10⁴ et 5 x 10⁴ (Tableau 1). La titration des unités formant colonie bleue par millilitre (BCFU/ml) du surnageant viral de Ψ2-MloxPL.1 et Ψ2-MloxPL.2 est respectivement de 3 et 6, ce qui correspond à un rapport approximatif de 1 colonie bleue sur 10⁴ intégrations. Comme nous l'avons décrit précédemment, ces provirus fonctionnent comme des pièges à promoteur, et seulement une intégration sur 10⁴ exprime le gène rapporteur dans la lignée cellulaire fibroblastique NIH3T3.

Les structures de l'ADN des provirus MloxPL intégrés sont schématisées à la figure 1b. Dans les figures du présent texte, les positions des LTR et des sites de restriction Bcll et les tailles des fragments sont indiquées (x indique le fragment Bdl de taille aléatoire du bras gauche et y le fragment Bcll de taille aléatoire du bras droit. P indique une sonde LacZ radiomarquée au 32p. Ces structures ont ensuite été analysées par hybridation par "Southern blot" dans 6 clones NIH 3T3 indépendants. Les résultats de cette analyse sont présentés à la figure 1d. Tous les clones contiennent un provirus dans lequel les séquences à l'intérieur de la région U3 de la 3'LTR ont été dupliquées dans la 5'LTR ; la digestion par l'endonucléase *Bcl* I génère le fragment attendu de la part des provirus avec deux LTR contenant *PhleoLacZ* (l'enzyme de restriction *Bcl* I a un site de reconnaissance dans chaque séquence *LacZ* dans les LTR). L'hybridation par "Southern blot", utilisant *LacZ* comme sonde, montre un fragment de clivage de 6 kpb démontrant une duplication fidèle de la région U3. Les deux bandes supplémentaires de tailles variables représentent des fragments qui s'étendaient des sites *Bcl* I dans le provirus jusqu'à l'ADN cellulaire flanquant. Les deux bandes supplémentaires montrent qu'il y a une intégration provirale par clone, et leurs tailles variables confirment que chaque lignée cellulaire était un clone indépendant.

### Exemple 2 : Cotransfection de lignées cellulaires avec les Plasmides pMloxPL et pMCl-Cre

Nous avons examiné si la duplication *loxP* par l'intermédiaire de la LTR peut être recombinée par la recombinase Cre. Nous avons utilisé la lignée cellulaire clonale NIH3T3MloxPL.1 pour cibler une recombinaison avec la protéine Cre. Nous avons cotransfecté dans cette lignée cellulaire le vecteur d'expression pMC1-Cre avec le vecteur de sélection pUSVneo. La structure du provirus après la recombinaison induite par Cre est illustrée à la figure 1c.

L'ADN de 24 clones résistant à la néomycine a été analysé par hybridation par "Southern blot". La détection des provirus recombinés a été réalisée en analysant les digestions de *Bcl* I. L'ADN restreint des clones a été sondé avec un *LacZ* radiomarqué au 32p. Les résultats de cette analyse sont illustrés à la figure 1e. La structure parentale du provirus montre une bande de 6 kpb correspondant à la duplication fidèle de la séquence U3 contenant *LacZ* et deux bandes supplémentaires de 8 et 2 kpb (décrites ci-dessus (Fig. 1b & d)). L'analyse de l'ADN des clones résistant à la néomycine montre 5 clones sur 24 dans lesquels la bande de 6,0 kpb a été éliminée (Fig. 1e). Les bandes de 8 et 2 kpb correspondant à l'ADN cellulaire flanquant le provirus étaient toujours présentes, montrant ainsi que le site d'intégration proviral n'a pas été réarrangé. Ce résultat suggère que l'expression du gène *cre* dans une cellule contenant le provirus MloxPL peut conduire à une recombinaison fréquente des deux sites *loxP* inclus dans les LTR.

Pour tester l'efficacité de la recombinaison par l'intermédiaire de *Crelox,* nous avons également analysé l'ADN de 25 clones résistant au G418 par hybridation par "Southern blot" afin de tester la présence de la séquence *cre*). L'ADN a été digéré avec l'endonucléase de restriction *Xho* I et sondé avec un ADN *cre* pleine longueur radiomarqué au 32p. Les résultats de cette analyse sont illustrés à la figure 1e. Trois des cinq clones recombinés en *loxP* montrent une bande s'hybridant avec la sonde *cre* de 1,9 kpb suggérant que l'unité d'expression *cre* pleine longueur de pMC1-Cre est présente. Un des cinq clones recombinés en *loxP* ne montrait pas de bande s'hybridant avec la sonde *cre.* Un clone recombiné en *loxP* montre une bande de 5 kpb résultant d'un réarrangement du plasmide pMC1-*Cre.* Quatre autres clones montrant une bande de 1,9 kpb s'hybridant avec *cre* ne présentent pas de recombinaison dans le provirus intégré MloxPL. Nous n'avons pas détecté la présence d'ADN *cre* dans les 16 clones restants. Ce résultat montre que l'efficacité de la recombinaison induite par la présence du plasmide pMC1-Cre n'est pas absolue dans les lignées cellulaires transfectées stables, où seulement 50% des sites *loxP* sont recombinés. Ce résultat suggère également que la recombinaison entre les deux sites *loxP* peut être obtenue par l'expression éphémère de pMC1-Cre (Tableau 2).

### Exemple 3 - Infection des cellules exprimant l'enzyme Cre avec MloxPL.

Nous avons produit une lignée cellulaire NIH3T3 contenant le plasmide pMC1-Cre, par cotransfection des plasmides pMC1-Cre et pUSVneo. Des clones ont été sélectionnés dans un milieu contenant du G418 et leur ADN a été analysé après digestion avec une endonucléase de restriction *Xho* I par hybridation par "Southern blot". La présence du gène *Cre* a été détectée par hybridation avec une sonde d'ADN *cre* pleine longueur radiomarquée au 32p. Dix des 12 clones résistant au G418 analysés ont révélé la présence de la séquence Cre sous un nombre variable de copies (allant approximativement de 1 à 10 copie(s))(données non illustrées).

Nous avons choisi le clone NIH3T3Cre.1 contenant approximativement 10 copies du plasmide pMC1-Cre à des fins d'analyse complémentaire. Nous avons infecté le clone NIH3T3Cre.1 avec le virus MloxPL. Les clones ont été isolés par dilution limitante.

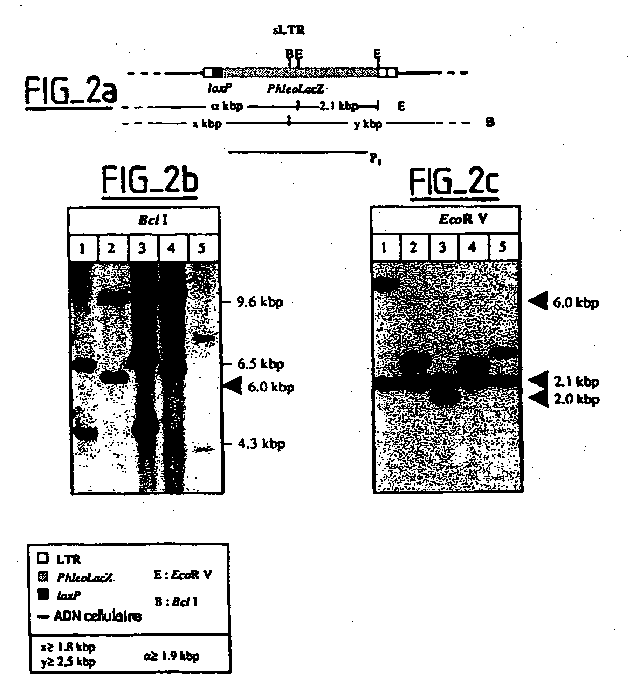

La structure du provirus résultant de l'infection du rétrovirus MloxPL et de la recombinaison induite par Cre est illustrée à la figure 2a. La structure du vecteur intégré est une LTR solitaire (sLTR). Il manque une LTR et toutes les séquences situées entre les deux LTR dans les provirus intégrés (Fig. 2a & c)

. La structure de l'ADN des provirus MloxPL a été analysée par hybridation par "Southern blot" après digestion par l'endonucléase de restriction *Bcl* I de leur ADN cellulaire afin de détecter la présence, ou l'absence, de LTR dupliquées dans la structure provirale. Tel que démontré à la figure 2b, l'analyse des 12 clones résultant de l'infection de NIH3T3Cre.1 par MloxPL a montré l'absence dans tous les clones de la bande de 6 kpb indicatrice de la duplication.

Pour démontrer l'absence de la 5'LTR dans le provirus, une restriction de l'ADN par *Eco*R V a été réalisée. L'hybridation par "Southern blot" de l'ADN de NIH3T3Cre.1.MloxPL, restreint par l'endonucléase *Eco*R V en utilisant une sonde *LacZ* radiomarquée (Fig. 2a & c), montre la présence d'une bande de 2,1 kpb et d'une bande de taille aléatoire. L'absence d'une bande de 3,9 kpb démontre l'absence d'une 5'LTR.

### Exemple 4 - Construction du vecteur rétroviral pMcreloxPL et transfection de lignées cellulaires

### a) Construction du plasmidepMcreloxPL

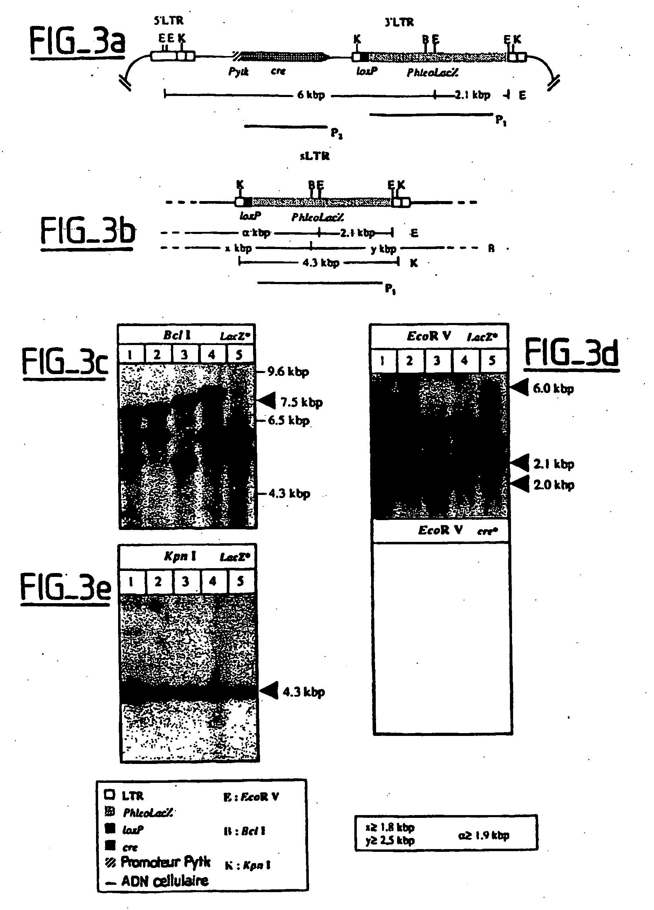

La structure de rétrovirus utilisé dans le présent exemple est illustrée à la Fig. 3a. pMCreloxPL résulte de l'insertion du gène *cre* de 1.3 kpb fusionné avec une localisation nucléaire du gros antigène T du virus simien 40, entre les deux LTR du plasmide pMloxPL. Le gène *cre* est sous le contrôle transcriptionnel du promoteur du gène de la thymidine kinase (*tk*) du virus herpès simplex flanqué par une duplication de l'activateur à distance du virus du polyome mutant PYF441 avec des adaptateurs de liaison au niveau du site Pstl de pMloxPL. La séquence *Cre* est dans la même orientation que le génome viral. Le plasmide pMcreloxPL a été déposé à la CNCM le 13 juin 1995 no I-1599.

### b) Transfection de lignées cellulaires avec le plasmide pMcreloxPL

Des lignées cellulaires produisant le virus ont été générées en cotransfectant le plasmide pMCreloxPL avec le plasmide de sélection pUSVneo dans la lignée cellulaire d'encapsidation Ψ2. Après sélection des cellules Ψ2 transfectées dans un milieu contenant du G418, des clones individuels ont été sélectionnés pour la production d'un rétrovirus exprimant *LacZ.* Un clone a produit un virus infectieux ; ce clone a été appelé Ψ2-MCreloxPL.1. Dans Ψ2-MCreloxPL.1, le plasmide pMCreloxPL a été intégré en une seule copie dans le génome cellulaire hôte (données non illustrées). Les lignées cellulaires productrices de Ψ2-MCreloxPL.1 produisent 1 x 10⁴ virus infectieux par millilitre ; la présence du gène *LacZ* est détectée par PCR (Tableau 1). La titration des unités formant colonie bleue par millilitre (BCFU/ml) du surnageant viral de Ψ2-MCreloxPL.1 est de 3 BCFU/ml, ce qui correspond comme prévu à une diminution approximative de 10⁴ par rapport à la titration par PCR. La production de Ψ 2-MCreloxPL était d'une efficacité basse comparée à la production de Ψ2-MloxPL, un seul clone producteur ayant été récupéré après plusieurs expériences de cotransfection. Ce clone avait intégré une seule copie de la construction plasmidique virale. Nous en avons déduit que les cellules qui intègrent plus d'une copie du plasmide ne peuvent pas être récupérées en raison de la présence de l'activité recombinase qui modifie les transgènes intégrés.

### c) Structure provirale de MCreloxPL

L'isolation de cellules NIH 3T3 infectées avec MCreloxPL a été réalisée par dilution limitante, après infection à une multiplicité de 0,5 particule virale par cellule. La structure du provirus résultant de l'infection du rétrovirus pMCreloxPL est illustrée à la figure 3b. Dans cette figure, a indique le fragment de taille aléatoire du bras gauche.

La structure de l'ADN des provirus intégrés a été analysée par hybridation par "Southern blot" dans 6 clones NIH3T3MCreloxPL indépendants. L'analyse par "Southern blot" de l'ADN proviral restreint par les endonucléases *Bcl* I et détecté avec une sonde *LacZ* radiomarquée a généré deux fragments de tailles variables, et ces clones n'ont pas généré de bande de 7,5 kpb supplémentaire (Figure 3c). L'absence de ce fragment supplémentaire de 7,5 kpb démontre la présence d'une seule LTR contenant *PhleoLacZ.* Pour établir en plus que la structure provirale correspond à une seule LTR, une analyse supplémentaire a été entreprise. Pour démontrer définitivement l'absence d'une 5'LTR dans le provirus, une restriction par *Eco*R V de l'ADN cellulaire a été réalisée. L'hybridation par "Southern blot" de l'ADN de NIH3T3MCreloxPL, restreint par l'endonucléase *Eco*R V en utilisant une sonde *LacZ* radiomarquée (Fig. 3d), montre la présence d'une bande de 2,1 kpb et d'une bande de taille aléatoire. L'absence d'une bande de 6 kpb démontre l'absence d'une 5'LTR. Ce "blot" a alors été hybridé avec une sonde *cre* radiomarquée. L'absence d'une bande dans l'ADN de NIH3T3MCreloxPL montre que l'ADN proviral est dépourvu du gène *cre* (Fig. 3d). Finalement, pour établir que la structure de la LTR restante n'était pas réarrangée, l'ADN de NIH3T3MCreloxPL a été restreint par l'endonucléase *Kpn* I et sondé avec un ADN de *LacZ* radiomarqué. Un fragment de 4,3 kpb a été observé dans tous les cas, montrant que la LTR *PhleoLacZ* était de la taille attendue (Fig. 3e). En conséquence, tous les clones isolés contenaient un provirus intégré avec une seule LTR non-réarrangée.

**TABLEAU 1. Titres des lignées cellulaires Ψ2 MloxPL et MCreloxPL^{a}.**

| Clone | IP/ml^{b} (10⁴) | BCF/ml^{c} | Rapport^{d} (10⁻⁴) |
|---|---|---|---|
| Ψ2-MloxPL.1 | 2,5 | 3 | 1,2 |
| Ψ2-MloxPL.2 | 5 | 6 | 1,2 |
| Ψ2-MCreloxPL.1 | 1 | 3 | 3 |

| | | | |
|---|---|---|---|
| ^{a} Des cellules Ψ2 ont été transfectées respectivement avec soit pMloxPL+pUSVneo, soit pMCreloxPL+pUSVneo, des clones résistants au G418 ont été sélectionnés dans un milieu contenant du G418. Des stocks de virus ont été préparés en incubant 8 ml de milieu avec 5 x 10⁶ cellules de chaque clone résistant au G418, 5 x 10⁴ cellules NIH 3T3 ont été soumises à diverses dilutions de virus pendant 8 heures et ensuite clonées par dilution limitante ou testées pour leur activité β-galactosidase par coloration par X-gal. ^{b} Les titres ont été calculés par le rapport entre les clones infectés isolés après clonage et détectés par PCR (IP : particule infectieuse) et les clones non-infectés (PCR+/PCR-)/1 x 10⁵). BCFU est l'acronyme de "blue colony forming unit" (unité formant colonie bleue). Les BCFU sont des infections aboutissant à un piège du gène qui active le gène *PhleoLacZ* détecté par coloration par X-gal. ^{d}Le rapport est le nombre de BCFU par clone infecté. | | | |

**TABLEAU 2. Recombinaison loxP par l'intermédiaire d'une transfection pMC1-Cre.**

| Nombre de clones résistant au G418^{a} | Présence de pMC1-Cre^{b} | Recombinaison *loxP*^{c} |
|---|---|---|
| 3 | + | + |
| 1 | - | + |
| 1 | * | + |
| 4 | + | - |
| 16 | - | - |

| | | |
|---|---|---|
| ^{a} Clones résistant au G418 provenant du clone NIH3T3MloxPL.1 cotransfecté avec pMC1-Cre et pUSVneo. ^{b} Indique la présence de la séquence pMC1-Cre. (+) Indique la présence d'un fragment *Xho* I de 1,9 kpb correct issu de pMC1-Cre détecté avec une sonde *cre* radiomarquée au ³²P. (*) Indique la présence d'un fragment *Xho* I s'hybridant avec la sonde *cre* radiomarquée au ³²P, mais d'une taille incorrecte. (-) Indique l'absence du plasmide pMC1-Cre. Résultats de la recombinaison *loxP* par l'intermédiaire de Cre détectée par analyse par "Southern blot". | | |

### Bibliographie

1.l. Barker, D. D., H. Wu, S. Hartung, M. Breindt, and R. Jaenisch. 1991. Retrovirus-induced insertional mutagenesis: mechanism of collagen mutation in Mov13 mice. Mol. Cel. Biol. 11:5154-5163.
2. Choi, S. Y., and D. V. Faller. 1994. The long terminal repeats of a murine retrovirus encode a trans-activator for cellular genes. J. Biol. Chem. 269:19691-19694.
3. Choulika, A., A. Perrin, B. Dujon, and J. F. Nicolas. 1994. Site-specific induction of homologous recombination in mammalian cells by I-Sce I system of Saccharomyces cerevisiae. Submitted to Science
4. Choulika, A., A. Perrin, B. Dujon, and J. F. Nicolas. 1995. Induction of homologous recombination in mammalian chromosomes by using the I-Sce I system of Saccharomyces cerevisioe. Mol. Cel. Biol. 15:1968-1973.
5. Chu, T.-H. T., and R. Dornburg. 1995. Retroviral vector particles displaying the antigen-binding site of an antibody enable cell-type-specific gene transfer. J. Virol. 69:2659-2663.
6. Church, G. M., and W. Gilbert. 1984. Genomic sequencing. Proc. Natl. Acad. Sci. USA 81:1991-1995.
7. Eychene, A., C. Bechade, M. Marx, D. Laugier, P. Dezelee, and G. Calothy. 1990. Molecular and biological properties of c-mil transducing retroviruses generated during passage of Rous-associated virus type 1 in chicken neuroretina cells. J Virol 64:231-8.
8. Felder, M. P., A. Eychene, J. V. Barnier, I. Calogeraki, G. Calothy, and M. Marx. 1991. Common mechanism of retrovirus activation and transduction of c-mil and c-Rmil in chicken neuroretina cells infected with Rous-associated virus type 1. J Virol 65:3633-40.
9. Feuer, G., M. Taketo, R. C. Hanecak, and H. Fan. 1989. Two blocks in Moloney murine leukemia virus expression in undifferentiated F9 embryonal carcinoma cells as determined by transient expression assays. J. Virol. 63:2317-2324.
10. Flanagan, J. R., K. G. Becker, D. L. Ennist, S. L. Gleason, P. H. Driggers, B. Z. Levi, E. Appella, and K. Ozato. 1992. Cloning of a negative transcription factor that binds to upstream conserved region of Moloney murine leukemia virus. Mol. Cell Biol. 12:38-44.
11. Gama Sosa, M. A., D. H. Rosas, R. DeGasperi, E. Morita, M. R. Hutchinson, and R. Ruprecht. 1994. Negative regulation of the 5' long terminal repeat (LTR) by the 3' LTR in the murine proviral genome. Mol. Cel. Biol. 68:2662-2670.
12. Graham, F. L., and A. J. van der Eb. 1973. A new technique for the assay of infectivity of human adenovirus 5 DNA. Virology 52:456-467.
13. Gu, H., Y. R. Zou, and K. Rajewsky. 1993. Independent control of immunoglobin switch recombination at individual switch regions evidenced through Cre-loxP-mediaied gene targeting. Cell. 73:1155-1164.
14. Hanley, T., and J. P. Merlie, 1991. Transgene detection in unpurified mouse tail DNA by polymerase chain reaction. BioTechnics. 10:56.
15. Hantzopoulos, P. A., B. A. Sullenger, G. Ungers, and E. Gilboa. 1989. Improved gene expression upon transfer of the adenosine deaminase minigene outside the transcriptional unit of a retroviral vector. Proc. Natl. Acad. Sci. USA 86:3519-3523.
16. Hoeben, R. C., A. A. Migchielsen, d. J. R. van, O. H. van, and d. E. A. van. 1991. Inactivation of the Moloney murine leukemia virus long terminal repeat in murine fibroblast cell lines is associated with methylation and dependent on its chromosomal position. J Virol 65:904-12.
17. Joyner, A. L. 1991. Gene targeting and gene trap screens using embryonic stem cells : New approaches to mammalian developement. BioEssays 13:649-656.
18. Kasahara, N., A. M. Dozy, and Y. W. Kan. 1994. Tissus-specific targeting of retroviral vectors through ligand-receptor interactions. Science 266:1373-1376.
19. Kilby, N. J., M. R. Snaith, and J. A. II. Murray. 1993. Site-specific recombinases : tools for genome engineering. Reviews 9:413-421.
20. Linney, E., B. Davis, J. Overhauser, E. Chao, and H. Fan. 1984. Non-function of Moloney murine leukaemia virus regulatory sequence in F9 embryonal carcinoma cells, Proc. Natl. Acad. Sci. USA 84:3748-3752.
21. Lochet, M., M. Aboud, and R. M. Flugel. 1993. Increase in the basal transcriptional activity of the human foamy virus internal promoter by the homolgous long terminal repeat promoter in cis. NAR 21:4226-4230.
22. Loh, T. P., L. L. Sievert, and R. W. Scott. 1988. Negative regulation of retrovirus expression in embryonal carcinoma cells mediated by an intragenic domain. J. Virol. 62:4086-4095.
23. Loh, T. P., L. L. Sievert, and R. W. Scott. 1990. Evidence for a stem cell-specific repressor of moloney murine leukemia virus expression in embryonal carcinoma cells. Mol. Cell. Biol. 10:4045-4057.
24. Loh, T. P., L. L. Sivert, and R. W. Scott. 1987. Proviral sequences that restrict retroviral expression in mouse embryonal carcinoma cells, Mol. Cell. Biol. 7:3775-3784.
25. Mann, R., R. C. Mulligan, and D. Baltimore, 1983. Construction of a retrovirus packaging mutant and its use to produce helper-free defective retrovirus, Cell. 33:153-160.
26. Mansour, S. L., K. R. Thomas, and C. M.R. 1988. Disruption of the proto-oncogene int-2 in mouse embryo-derived stem cells : a general strategy for targeting mutations to non-selectable gènes. Nature 336:348-352.
27. Miller, A. D., D. R. Trauber, and C. Buttimore. 1986. Factors involved in the production of helper virus-free retrovirus vector, Somatic Cell. Mol. Genet. 12:175-183.
28. Morgan, R. A., L. Couture, O. Elroy-Stein, J. Ragheb, B. Moss, and W. F. Anderson. 1992. Retroviral vectors containing putative internal ribosome entry sites: developement of a polycistronic gene transfer and applications to human gene therapy. N.A.R. 20:1293-1299.
29. Nicolas, J. F., and C. Bonnerot. 1993. Répression et activation des rétrovirus murins dans les cellules totipotentes. Médecine/Sciences 9:191-197.
30. Nicolas, J. F., and J. Rubenstein. 1987. Retroviral vectors, p. 493-512. In E. Biotechnology series - Julian E. Davies (ed.), Vectors : A survey of molecular cloning vectors and their uses. Butterworths, Boston London Durban Singapore Sydney Toronto Wellington.
31. Pear, W. S., G. P. Nolan, M. L. Scott, and D. Baltimore. 1993. Production of high-titer helper-free retroviruses by transient transfection. Proc. Natl. Acad. Sci. USA. 90:8392-8396.
32. Peters, G., A. E. Lee, and C. Dickson. 1986. Concerted activation of two potential proto-oncogenes in carcinomas induced by mouse mammary tumour virus. Nature 320:628-631.
33. Petersen, R., G. Kempler, and E. Barklis. 1991. A stem cell-specific silencer in the primer-binding site of a retrovirus. Mol. Cel. Biol. 11:1214-1221.
34. Pulsinelli, G. A., and H. M. Temin. 1991. Characterization of large deletions occurring during a single round of retrovirus vector replication : novel deletion mechanism involving errors in strand transfer. J. Virol. 65:4786-4797.
35. Reddy, S., J. V. DeGregori, H. Von Melchner, and H. E. Ruley. 1991. Retrovirus promoter-trap vector to induce LacZ gene fusions in mammalian cells. J. Virol. 65:1507-1515.
36. Reddy, S., H. Rayburn, V. M. H., and R. H. E. 1992. Fluorescence-activated sorting of totipotent embryonic stem cells expressing developmentally regulated lacZ fusion genes. Proc. Natl. Acad. Sci. 89:6721-6725.
37. Rubenstein, J., J. F. Nicolas, and F. Jacob. 1984. Construction of a retrovirus capable of transducing and expressing genes in multipotential embryonic cells. Proc. Natl. Acad. Sci. USA 81:7137-7140.
38. Sanes, J., J. Rubenstein, and J. F. Nicolas. 1986. Use of a recombinant retrovirus to study post-implantation cell lineage in mouse embryos. EMBO J. 5:3133-3142.
39. Sauer, B. 1987. Functional expression of the cre-lox site-specifie recombination system in the yeast Saccharomyces cerevisiae. Mol. Cel. Biol. 7:2087-2096.
40. Sauer, B., and N. Henderson. 1988. Site-specific DNA recombination in mammalian cells by the Cre recombinase of bacteriophage P1. Proc. Natl. Acad. Sci. USA 85:5166-5170.
41. Savatier, N., D. Rocancourt, C. Bonnerot, and J.-F. Nicolas. 1989. A novel system for screening antiretroviral agents. J. Virol. 26:229-236.
42. Shafer, G. E., D. W. Emery, K. Gustafsson, S. Germana, W. F. Anderson, D. H. Sachs, and C. LeGuern. 1991. Expression of a swine class II gene in murine bone marrow hematopietic cells by retroviral mediated gene transfer. Proc. Natl. Acad. Sci. USA 88:9760-9764.
43. Sternberg, N., B. Sauer, R. Hoess, and K. Abremski. 1986. Bacteriophage P1 cre gene and its regulatory region. Evdence for multiple promoter and for regulation by DNA methylation. J. Mol. Biol. 187:197-212.
44. Stevenson, M., S. Haggerty, C. A. Lamonica, C. M. Meier, S. K. Welch, and A. J. Wasiak. 1990. Integration is not necessary for expression of human immunodeficiency virus type 1 protein products. J. Virol. 64:2421-2425.
45. Stuhlmann, H., and P. Berg. 1992. Homologous recombination of copakaged retrovirus RNAs during revers transcription. J. Virol. 66:2378-2388.
46. Swanstrom, R., R. C. Parker, H. E. Varmus, and J. M. Bishop. 1983. Transduction of a cellular oncogene : the genesis of Rous sarcoma virus. Proc. Natl. Acad. Sci. USA 80:2519-2523.
47. Takeuchi, Y., G. Simpson, R. G. Vile, R. A. Weiss, and M. K. Collins. 1992. Retroviral pseudotypes produced by rescue of a Moloney murin leukemia virus vector by C-type, but not D-type, retrovirus. Virology 186:792-794.
48. Tramblay, P. J., C. A. Kozak, and P. Jolicoeur. 1992. Identification of a novel gene, Vin-1, in murine leukemia virus-induced T-cell leukemias by provirus insertional mutagenesis. J. Virol. 66:1344-1353.
49. Trusko, S. P., E. K. Hoffman, and D. L. George. 1989. Transcriptional activation of cKi-ras proto-oncogene resulting from retroviral promoter insertion. N.A.R. 17:9259-9265.
50. Varcla-Echavarria, A., C. M. Prorock, Y. Ron, and J. P. Dougherty. 1993. High rate of genetic rearrengement during replication of Moloney murin leukemia virus-based vector. J. Virol. 67:6357-6364.
51. von Melchner, H., and H. E. Ruley. 1989. Identification of cellular promoters by using a retrovirus promoter trap. J. Virol. 63:3227-3233.
52. Weiss, R., N. Teich, H. Varmus, and J. Coffin. 1985. RNA tumor viruses., p. 1222. (ed.), Molecular Biology of tumor viruses. Cold Spring Habor Laboratory.,
53. Yee, Y. K., J. C. Moores, D. J. Jolly, J. A. Wolff, J. G. Respess, and T. Friedmann. 1987. Gene expression from transcriptionally disabled retroviral vectors. Proc. Natl. Acad. Sci. USA 84:5197-5201.

## Revendications

1. Vecteur rétroviral **caractérisé en ce qu'**il comprend un polynucléotide comprenant
(a) la séquence LTR3' et LTR5' d'un retroélément naturel ou synthétique ; et
(b) une séquence d'insertion comprenant une séquence de nucléotides d'intérêt ainsi que, pour un système de recombinaison donné, une unique séquence de reconnaissance d'une recombinase,
ladite séquence d'insertion étant insérée dans ladite LTR3' ou ladite LTR5', de façon à permettre l'insertion de ladite séquence d'intérêt dans le génome d'une cellule hôte, sans que soient insérées des séquences provirales qui ne sont plus nécessaires après l'intégration de la séquence d'intérêt dans le génome de la cellule hôte,
l'expression de la recombinase reconnaissant ladite séquence de reconnaissance n'étant pas associée directement audit vecteur.

2. Vecteur rétroviral selon la revendication 1, **caractérisé en ce que** ladite séquence d'insertion du polynucléotide est incorporée dans la région U3 de la LTR3', U5 de la LTR5', ou R de la LTR.

3. Vecteur rétroviral selon l'une quelconque des revendications 1 à 2, **caractérisé en ce que** le rétroélément est une séquence d'ADN rétroviral.

4. Vecteur rétroviral selon l'une quelconque des revendications 1 à 3, **caractérisé en ce que** ladite séquence de reconnaissance pouvant être reconnue par une recombinase est située en amont de ladite séquence de nucléotides d'intérêt.

5. Vecteur rétroviral selon l'une quelconque des revendications 1 à 3, **caractérisé en ce que** ladite séquence de reconnaissance pouvant être reconnue par une recombinase est située en aval de ladite séquence de nucléotides d'intérêt.

6. Vecteur rétroviral selon l'une quelconque des revendications précédentes, **caractérisé en ce que** la séquence de reconnaissance comprend le site de reconnaissance LoxP.

7. Vecteur rétroviral selon l'une quelconque des revendications précédentes, **caractérisé en ce que** la séquence de nucléotides d'intérêt comprend une séquence codant pour une protéine ou un ARN d'intérêt, particulièrement un ARN antisens ou une séquence ribozyme.

8. Vecteur rétroviral selon l'une quelconque des revendications précédentes, **caractérisé en ce que** la séquence de nucléotides d'intérêt comprend en outre des éléments de régulation.

9. Vecteur rétroviral selon l'une quelconque des revendications 1 à 8, **caractérisé en ce que** ledit vecteur est un provirus de leucémie murine de Moloney défectif comprenant ladite séquence de reconnaissance dans sa région U3 du LTR3', U5 du LTR5' ou R.

10. Méthode d'introduction d'un gène dans une cellule hôte en culture, ladite méthode étant **caractérisée en ce qu'**elle comprend l'infection de ladite cellule hôte par un vecteur rétroviral selon l'une quelconque des revendications 1 à 9.

11. Méthode d'expression d'une molécule codée par une séquence d'intérêt dans une cellule hôte en culture, ladite méthode étant **caractérisée en ce qu'**elle comprend :
- l'infection de ladite cellule hôte par un vecteur rétroviral selon l'une quelconque des revendications 1 à 9 comprenant ladite séquence de nucléotides d'intérêt ;
- la croissance de ladite cellule hôte dans des conditions permettant l'expression de ladite séquence de nucléotides d'intérêt; et
- l'obtention de la molécule désirée.

12. Méthode d'expression selon la revendication 11 comprenant en outre la transfection de la cellule hôte par un plasmide comprenant une séquence de nucléotides codant pour une recombinase susceptible de reconnaître la séquence de reconnaissance.

13. Méthode d'expression selon la revendication 11, **caractérisée en ce que** la cellule hôte exprime constitutivement ou par induction une recombinase susceptible de reconnaître la séquence de reconnaissance.

14. Méthode d'expression selon la revendication 12 ou 13, **caractérisée en ce que** la séquence de nucléotides codant pour une recombinase code pour la protéine Cre.

15. Utilisation d'un vecteur rétroviral selon l'une quelconque des revendications 1 à 9, pour la fabrication d'une composition permettant la production par des cellules hôtes de protéines codées par une séquence de nucléotides d'intérêt.

16. Vecteur rétroviral selon l'une quelconque des revendications 1 à 9, pour utilisation dans le traitement médical d'un patient.

17. Composition pharmaceutique comprenant un vecteur rétroviral selon l'une quelconque des revendications 1 à 9 en combinaison avec un excipient pharmaceutique acceptable.

## Claims

1. Retroviral vector, **characterised in that** it comprises a polynucleotide comprising
(a) the LTR3' and LTR5' sequence of a natural or synthetic retroelement;
and
(b) an insertion sequence comprising a nucleotide sequence of interest as well as, for a given recombination system, a unique recombinase recognition sequence;
said insertion sequence being inserted into said LTR3' or said LTR5' so as to allow insertion of said sequence of interest into the genome of a host cell, without inserting proviral sequences which are no longer necessary after integrating the sequence of interest into the genome of the host cell,
the expression of the recombinase recognising said recognition sequence not being directly associated with said vector.

2. Retroviral vector according to claim 1, **characterised in that** said insertion sequence of the polynucleotide is incorporated into the U3 region of the LTR3', the U5 region of the LTR5' or the R region of the LTR.

3. Retroviral vector according to any one of claims 1 to 2, **characterised in that** the retroelement is a retroviral DNA sequence.

4. Retroviral vector according to any one of claims 1 to 3, **characterised in that** the recognition sequence which may be recognised by a recombinase is situated upstream of said nucleotide sequence of interest.

5. Retroviral vector according to any one of claims 1 to 3, **characterised in that** the recognition sequence which may be recognised by a recombinase is situated downstream of said nucleotide sequence of interest.

6. Retroviral vector according to any one of the preceding claims, **characterised in that** the recognition sequence comprises the LoxP recognition site.

7. Retroviral vector according to any one of the preceding claims, **characterised in that** the nucleotide sequence of interest comprises a sequence coding for a protein or an RNA of interest, particularly an antisense RNA or a ribozyme sequence.

8. Retroviral vector according to any one of the preceding claims, **characterised in that** the nucleotide sequence of interest further comprises regulation elements.

9. Retroviral vector according to any one of claims 1 to 8, **characterised in that** said vector is a defective Moloney murine leukaemia provirus comprising said recognition sequence in its U3 region of LTR3', in its U5 region of LTR5' or in its R region.

10. Method of introducing a gene into a host cell in culture, said method being **characterised in that** it comprises infecting said host cell with a retroviral vector according to any one of claims 1 to 9.

11. Method of expressing a molecule coded by a sequence of interest in a host cell in culture, said method being **characterised in that** it comprises:
- infecting said host cell with a retroviral vector according to any one of claims 1 to 9 comprising said nucleotide sequence of interest;
- growing said host cell under conditions allowing expression of said nucleotide sequence of interest; and
- obtaining the desired molecule.

12. Method of expression according to claim 11, further comprising transfecting the host cell with a plasmid comprising a nucleotide sequence coding for a recombinase capable of recognising the recognition sequence.

13. Method of expression according to claim 11, **characterised in that** the host cell expresses, constitutively or by induction, a recombinase capable of recognising the recognition sequence.

14. Method of expression according to claim 12 or 13, **characterised in that** the nucleotide sequence coding for a recombinase codes for the protein Cre.

15. Use of a retroviral vector according to any one of claims 1 to 9, for the manufacture of a composition allowing the production, by host cells, of proteins coded by a nucleotide sequence of interest.

16. Retroviral vector according to any one of claims 1 to 9, for use in the medical treatment of a patient.

17. Pharmaceutical composition comprising a retroviral vector according to any one of claims 1 to 9 combined with a pharmaceutically acceptable excipient.

## Patentansprüche

1. Retroviraler Vektor, der **dadurch gekennzeichnet ist, dass** er ein Polynucleotid umfasst, das
a) die Sequenz LTR3' und LTR5' eines natürlichen oder synthetischen Retroelements; und
b) eine Insertionssequenz, umfassend eine Nucleotidsequenz von Interesse sowie, für ein gegebenes Rekombinationssystem, eine einzelne Erkennungssequenz einer Rekombinase,
umfasst,
wobei die Insertionssequenz so in das LTR3' oder LTR5' inseriert ist, dass die Insertion der Sequenz von Interesse in das Genom einer Wirtszelle ermöglicht wird, ohne dass provirale Sequenzen inseriert werden, die nach der Integration der Sequenz von Interesse in das Genom der Wirtszelle nicht mehr erforderlich sind,
wobei die Expression der Rekombinase, die die Erkennungssequenz erkennt, nicht direkt mit dem Vektor in Zusammenhang steht.

2. Retroviraler Vektor gemäß Anspruch 1, der **dadurch gekennzeichnet ist, dass** die Insertionssequenz des Polynucleotids in die Region U3 von LTR3', die Region U5 von LTR5' oder die Region R von LTR eingefügt wird.

3. Retroviraler Vektor gemäß einem der Ansprüche 1 bis 2, **dadurch gekennzeichnet, dass** das Retroelement eine retrovirale DNA-Sequenz ist.

4. Retroviraler Vektor gemäß einem der Ansprüche 1 bis 3, **dadurch gekennzeichnet, dass** die Erkennungssequenz, die durch eine Rekombinase erkannt werden kann, stromaufwärts von der Nucleotidsequenz von Interesse liegt.

5. Retroviraler Vektor gemäß einem der Ansprüche 1 bis 3, **dadurch gekennzeichnet, dass** die Erkennungssequenz, die durch eine Rekombinase erkannt werden kann, stromabwärts von der Nucleotidsequenz von Interesse liegt.

6. Retroviraler Vektor gemäß einem der vorstehenden Ansprüche, **dadurch gekennzeichnet, dass** die Erkennungssequenz die Erkennungsstelle LoxP umfasst.

7. Retroviraler Vektor gemäß einem der vorstehenden Ansprüche, **dadurch gekennzeichnet, dass** die Nucleotidsequenz von Interesse eine Sequenz umfasst, die ein Protein oder eine RNA von Interesse codiert, insbesondere eine Antisense-RNA oder eine Ribozymsequenz.

8. Retroviraler Vektor gemäß einem der vorstehenden Ansprüche, **dadurch gekennzeichnet, dass** die Nucleotidsequenz von Interesse zusätzlich Regulationselemente umfasst.

9. Retroviraler Vektor gemäß einem der Ansprüche 1 bis 8, **dadurch gekennzeichnet, dass** der Vektor ein defektives Moloney Murine Leukemia Provirus ist, der die Erkennungssequenz in seiner Region U3 von LTR3', U5 von LTR5' oder R umfasst.

10. Verfahren zur Insertion eines Gens in eine Wirtszelle in Kultur, wobei das Verfahren **dadurch gekennzeichnet, dass** es die Infizierung der Wirtszelle durch einen retroviralen Vektor gemäß einem der Ansprüche 1 bis 9 umfasst.

11. Verfahren zur Expression eines durch eine Sequenz von Interesse codierten Moleküls in einer Wirtszelle in Kultur, wobei das Verfahren **dadurch gekennzeichnet ist, dass** es umfasst:
- Infizierung der Wirtszelle durch einen retroviralen Vektor gemäß einem der Ansprüche 1 bis 9, der die Nucleotidsequenz von Interesse umfasst;
- Wachstum der Wirtszelle unter Bedingungen, die die Expression der Nucleotidsequenz von Interesse erlauben; und
- Gewinnung des gewünschten Moleküls.

12. Verfahren zur Expression gemäß Anspruch 11, das ferner die Transfektion der Wirtszelle mit einem Plasmid umfasst, das eine Nucleotidsequenz umfasst, die eine Rekombinase codiert, die fähig ist, die Erkennungssequenz zu erkennen.

13. Verfahren zur Expression gemäß Anspruch 11, **dadurch gekennzeichnet, dass** die Wirtszelle konstitutiv oder induziert eine Rekombinase exprimiert, die fähig ist, die Erkennungssequenz zu erkennen.

14. Verfahren zur Expression gemäß Anspruch 12 oder 13, **dadurch gekennzeichnet, dass** die eine Rekombinase codierende Nucleotidsequenz das Protein Cre codiert.

15. Verwendung eines retroviralen Vektors gemäß einem der Ansprüche 1 bis 9 für die Herstellung einer Zusammensetzung, welche die Produktion von Proteinen, die von einer Nucleotidsequenz von Interesse codiert werden, in Wirtszellen erlaubt.

16. Retroviraler Vektor gemäß einem der Ansprüche 1 bis 9 zur Verwendung in der medizinischen Behandlung eines Patienten.

17. Arzneimittel, umfassend einen retroviralen Vektor gemäß einem der Ansprüche 1 bis 9 in Verbindung mit einem pharmazeutisch verträglichen Trägerstoff.
